# EUROPEAN PATENT APPLICATION

(11) **EP 3 431 109 A1**
(43) Date of publication of application: **23.01.2019**
(21) Application number: 18382185.9
(22) Date of filing: 21.03.2018
(51) Int. Cl.: A61L 2/20, A46B 17/06, A47L 13/51, A61L 2/26

(54) **OBJECTS DISINFECTION DEVICE**

(30) Priority: 30.03.2017 ES 201700317 U
(71) Applicant: Franco Marin, Daniel Hector, 30509 Murcia (ES)
(72) Inventor: Franco Marin, Daniel Hector, 30509 Murcia (ES)

(57) **Abstract**

Objects disinfection device, which comprises a first body with a lower Surface, a lateral Surface and an open upper surface, such that define one first chamber suitable for containing a disinfecting product; a second body that comprises a lower surface with a plurality of holes, a lateral surface and one open upper surface, such that define a second chamber suitable to contain objects; where the second body is placed on the first and comprises watertight coupling means in the profile of the upper surface of the same, being the dimensions of the lower surface of the second body smaller or equal than the ones of the upper surface of the first; one impermeable and breathable lamina of separation between the first chamber and the lower surface of the second body, fixed to the same with holding means, and; at least one watertight upper lid on the upper area of the second body.

## Description

### Scope of the invention.

The present invention corresponds to the technical field of the objects disinfection devices, being these objects cleaning cloths, dishcloth, kitchen sponges and other cleaning devices as brushes, combs and other personal care devices, with the goal of avoiding the risk of infection by the use of the same.

### Background of the invention.

Currently, there are numerous studies which prove that the sponges, cleaning cloths, dishcloths used in the kitchen or other places in the house, are a focus of infection due to the appearance and development of bacteria and microorganisms.

These cleaning devices during its use collect organic material of all kind, which combined with a wet environment and the room temperature, are the idyllic ground for the production and growing of certain microorganisms and bacteria, which are a threat for the people's health.

The most common methods used for the disinfection of these kind of elements are soaking with some disinfection liquid the sponges and dishcloths, heat them in hot water, putting the in the dishwasher, in the microwave, etc. These methods are effective if they are correctly done, but very cumbersome and uneasy, and with a high risk of splashes or that the disinfection product come into contact with the skin, with the risk this means.

Furthermore, to be sure such sponges and dishcloths are free of dangerous microorganisms it would be necessary to disinfect with these methods after each use, which is very impractical. The usual practice is not disinfecting continuously the sponges, cleaning cloths, dishcloths, with the subsequent risk of contamination and infections which are the origin of diseases.

As an example of the state of art we can mention the reference documents ES2161185 and ES2356646.

The reference document ES2161185 defines an object disinfection device, consisting of a container to storage the objects to disinfect, and coupling such container in a separated way to an ozone generator group, with inclusion between both of an element with multiple tubes which determine outlets of ozone projections distributed inside the container, to project the ozone directly on the objects to disinfect.

This device uses the ozone as a disinfection media, and it is intended for objects of food, as baby bottles and teats. Furthermore, the prize of ozone and the difficulty to obtain this product as disinfectant, for common households, means and additional inconvenient due that this device works when it's connected to that end, and not for a continuous use.

This operation method is suitable in the case of elements as teats and baby bottles, in which once disinfected can be kept in perfect conditions until the next use, but in the case of sponges, dishcloths and cloths, are elements which are very frequently used and are always damp and in continuous contact with microorganisms, so they need a disinfection in continuous, that keep these object disinfected all the time, not only when the device is connected.

The reference document ES2356646 defines a disinfection device using a disinfection cleaning liquid for the care of health and similar, where the disinfection device comprises a fluid system to supply the fluid to a chamber, a first connection mobile part, at least partially with an active and inactive position, which adapts itself to a mobile porta-objects, where such porta-objects is placed in the chamber and has a second connection part which can be joined to such first connection part.

This disinfection device, apart from being intended for health care objects, it is quite complicated and cumbersome, so it is not appropriated for it use in common households for the disinfection of objects such as kitchen sponges, scouring sponges, cloths and dishcloths.

Nowadays there is not a device or container for kitchen sponges, cloths and dishcloths that have a disinfection system in continuous that furthermore doesn't have any danger for the users.

### Description of the invention.

The objects disinfection device, being such objects kitchen sponges, clothes, dishcloths and other cleaning tools as brushes, teeth brushes, combs and other personal care devices, which is the object of the present invention, comprises a first body made by a lower surface, a lateral surface and a upper open surface, such that define a first room suitable to contain a disinfection product. Furthermore, it presents a second body that comprises a lower surface with a plurality of holes, a lateral side and an open upper surface.

The disinfection device also comprises at least one watertight upper lid on the upper area of the second body, in a way that between such second body and the at least one upper lid, it is defined a second stay with at least one compartment suitable for the containing of objects.

Furthermore it comprises an impermeable and breathable lamina of separation between the first chamber and the lower surface of the second body, fixed to the same using holding means.

The second body is placed on the first body and comprises means of watertight coupling to the profile of the upper surface of the same, being the dimensions of the lower surface of the second body, smaller or equal than the one of the open upper surface of the first body.

According to one preferred embodiment, the coupling means of the second body on the first body are clip elements.

In this case and in one preferred embodiment, the disinfection device comprise furthermore, some opening and closing means from the second body, made by a first hinged element.

According to the preferred embodiment, when the lower surface of the second body has the same dimensions than the upper surface of the first body, the fixing means of the lamina are made by a watertight seal in the profile of the lower surface of the second body.

In another preferred embodiment, when the lower surface of the second body presents smaller dimensions than the upper surface of the first body, such second body comprise a lateral inner surface of connection with the coupling means and the fixing means of the lamina are made by an watertight seal placed in the lateral inner surface for the second body.

According to a different aspect, in a preferred embodiment, the second chamber comprises at least two separated compartments from each other.

In this case, and in a preferred embodiment, each compartments comprises an upper lid of closure of the same independent from each other.

According to a preferred embodiment, at least one upper lid is joined to the second body by means of a second hinged element.

According to a preferred embodiment, the disinfection device comprises a heating device of the disinfection product comprised by a resistance and one metallic plate on the same, placed in a watertight chamber in the lower side of the first body and with connection means of the resistance to a power supply in the lateral of such chamber.

In this way, when the resistance is connected to the power supply, this heats the metallic plate placed on the same and such metallic plate heats the disinfection products contained in the first chamber of the body.

According to a preferred embodiment, the disinfector device comprise reload means for the disinfectant product comprising an inner manifold communicating the first chamber of the first body with the second chamber of the second body and a closure cap of the upper ending of such manifold. In other embodiment of the invention, the reload is made separating the second body from the first body.

With the objects disinfection device proposed in this invention, it is obtained an important improvement in the state of art.

This is so because it is obtained an object disinfection device, where these objects can be sponges, kitchen sponges, dishcloths, cloths, and other cleaning tools commonly used in kitchens, which is easy to use, as to assemble and disassemble for its cleaning, and very practical on its diary use.

A great advantage of this disinfection device is that it is suitable to work with disinfecting products such as bleach, which is normally present in all the home kitchen, and which eases dramatically the use of this device.

This device works continuously generating steam that disinfect the products inside the device while they are not being used, so when they are in use, they are free of microorganisms and bacteria dangerous for our heath.

It is a safe device because it is hermetically closed and there is not possibility of accidental leakages of the disinfecting product. Furthermore, such disinfecting product does not contaminate because it is not in contact with the products to be disinfected and so, it is neither in contact with the organic material and microorganisms existing in the same, so it is possible a maximum exploitation of the product, with the subsequent economic saving.

Moreover, it is dramatically reduced the risk of stains in the clothes by splashes or the contact with hands, eyes, face...due that there isn't contact of the user with the disinfecting liquid, so it is reduced the possibility of accidents and risks for the health.

It is a disinfecting device simple, easy to use and very effective.

### Brief description of the drawings.

For the purpose of helping to a better understanding of the characteristics of the invention, according to an preferred embodiment of the same, it is given as an integral part of the description, some drawings, with illustrative character and not limiting , representing the following:
Figure 1.- Shows a cross-section of the object disinfection device, for a preferred embodiment of the invention.
Figure 2.- Show a profile section of the object disinfection device, for a preferred embodiment of the invention.
Figure 3.- Shows a perspective view from the top of the disinfection object device, for a preferred embodiment of the invention.

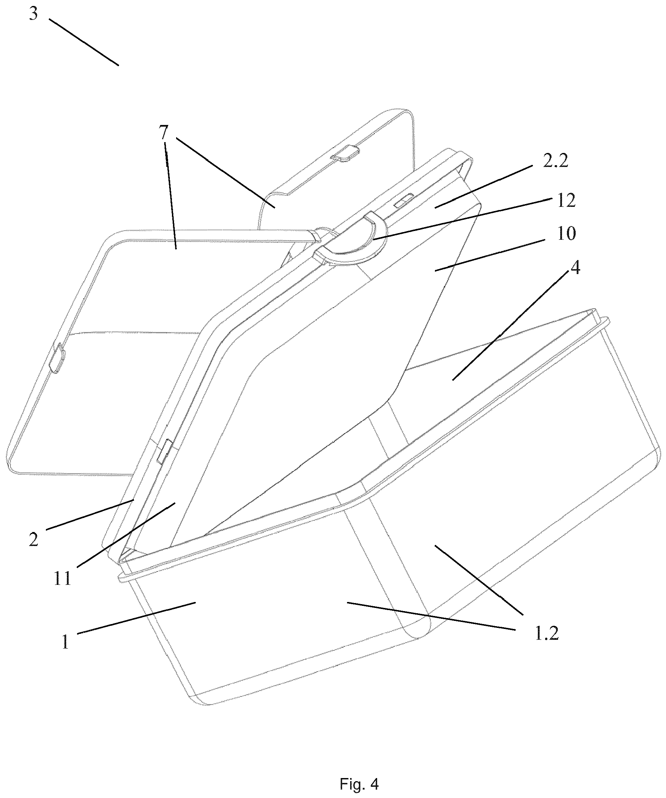

Figure 4.- Shows a perspective view from below of the objects disinfection device.

### Detailed description of the preferred embodiment of the invention.

In the view of the drawings, it can be observed as in a preferred embodiment of the invention, the object disinfection device (3) here introduced, being such objects in this case, sponges, cloths, dishcloths, comprises a first body (1) formed by a lower surface (1.1), a lateral surface (1.2) and a upper surface (1.3) open, such that define a first chamber (4) suitable to contain a disinfection product and, a second body (2) that comprise a lower surface (2.1) with a plurality of holes (5), a lateral surface (2.2) and a upper area (2.3) open.

As in can be observed in the Figures from 1 to 4, the second body (2) is placed on the first body (1) and presents watertight coupling means to the profile of the upper surface (1.1) of the same, where the dimensions of the lower surface (2.1) of the second body (2), are smaller or equal than the ones of the upper surface (1.1) of the first body (1).

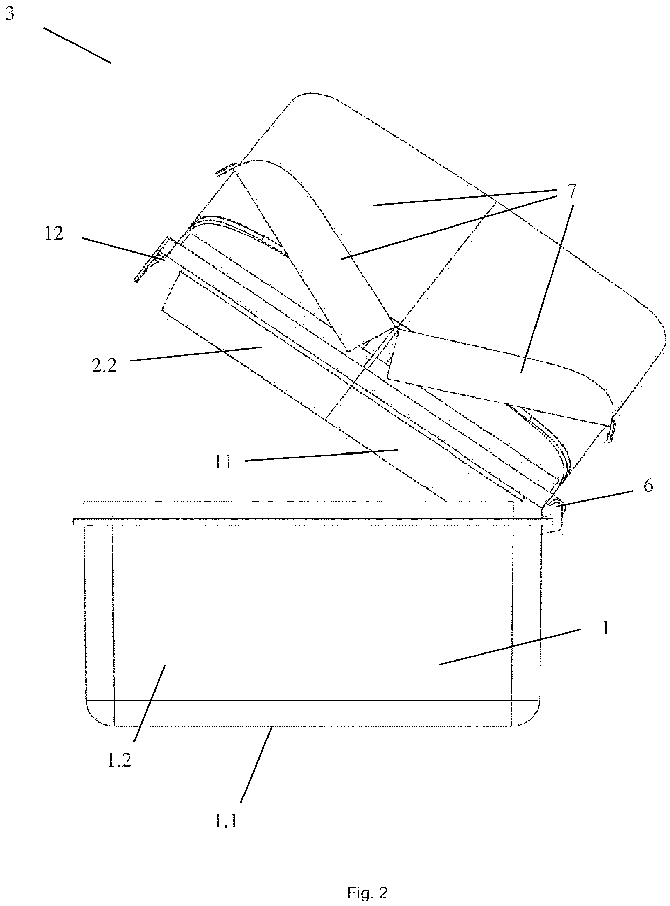

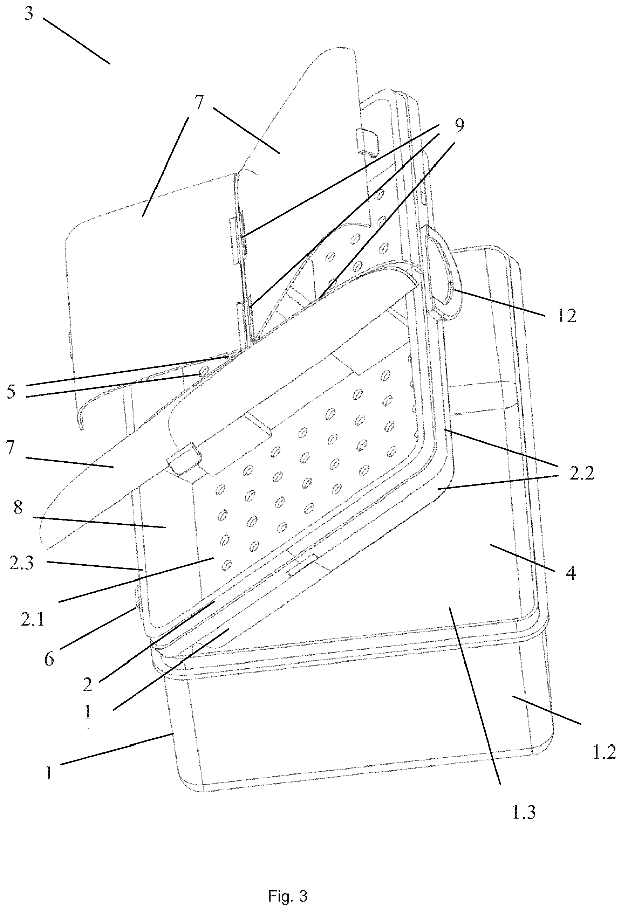

In this preferred embodiment of the invention, such coupling means are made by clip elements (12). Furthermore, this disinfection device (3) comprise open and closure mean from the second body, formed by a first hinged element (6) as observed in the figures 2 and 3.

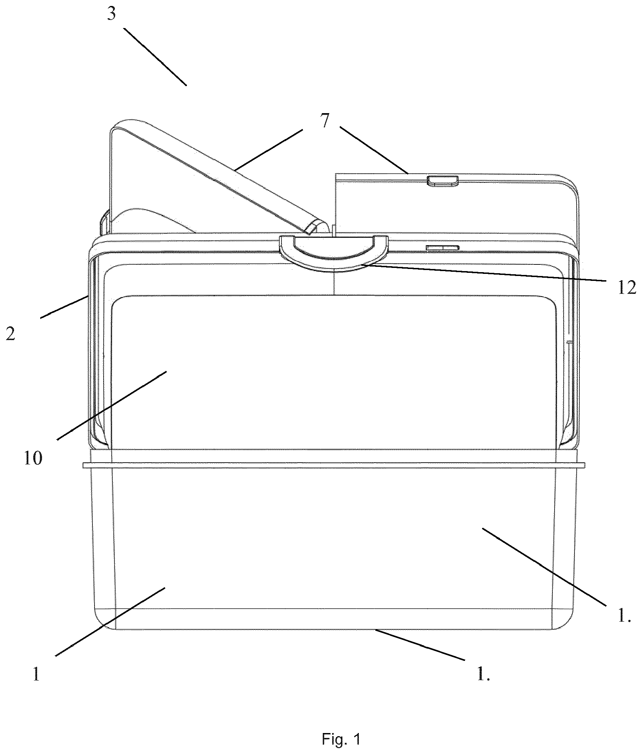

The disinfection device (3) comprises, as showed in Figure 1, at least one upper lid (7) watertight on the upper area (2.3) of the second body (2), so between the such second body (2) and such lid (7), is defined a second chamber (8) with at least one chamber suitable for the containing of objects. In this preferred embodiment of the invention, such second chamber (8) presents thee compartments separated among them, and each compartment present one upper lid (7) of closure independent from the same independently.

In this preferred embodiment of the invention, these upper closure lids (7) are joined to the second body (2) by means of hinged elements (9) that in this embodiment are disposed in the upper profile of the separation walls between such compartments, as can be observed in Figure 3.

This object disinfection device (3) comprises furthermore an impermeable and breathable lamina (10) of separation between the first chamber (4) and the lower surface (2.1) of the second body (2), fixed to the same using fixing means.

In this preferred embodiment of the invention, as is showed in the Figure 2, the lower Surface (2.1) of the second body (2) present smaller dimensions than the ones of the upper surface (1.3) of the first body (1). In this case, such second body (2) comprise an inner lateral surface (11) of connection of its lower surface (2.1), with the coupling means and, the fixing means of the lamina (10) are formed by a watertight seal placed in the inner lateral surface (11) of the second body (2).

Therefore, in this disinfection device (3) the disinfectant product is placed on the first chamber (4) existing in the first body (1) and it is hermetically sealed so much the second body (2) on the first body (1), as the three upper lids (7) of the device. The steam generated by the evaporation of the disinfection product, goes through the impermeable and breathable lamina (10) and, through the holes (5) existing in the lower surface (2.1) of the second body (2), reaches the second chamber (8) in which the objects to disinfect are.

The steam of the disinfecting product keep contained in such second chamber (8), generating a disinfecting atmosphere acting on the objects contained on it. In this preferred embodiment of the invention it is used bleach as disinfecting product, but on other embodiments and/ or according to the product to disinfect, other kind of disinfecting products can be used.

Furthermore, in this embodiment of the invention, to reload such disinfecting product the second body (2) is separated from the first body (1), to proceed comfortably in this way to the first chamber (4) and proceed to the reload of the same.

The already described embodiment only constitute an example of the present invention, thus, the details, terms and specific sentences used in the present memory must not be considered as limitative, but must be understood only as a base for the claims and as a representative base that supplies an understandable description as the enough information for the subject matter expert to apply the present invention.

With the disinfecting device here described important improvements are achieved with regard to the state of art.

It is a disinfection device comfortable and practical for the domestic use, furthermore of being economic, thanks that can be used with any disinfecting product, included the bleach, which is easy to find in any house.

It has an easy opening and closure, so it is easy to enter inside the device to pour the product and at the same time, the closure of the lid is watertight, avoiding the exit of the disinfecting steam and also watertight the closure between the first and second bodies, impeding on this way, possible accidents due to leakage. The impermeable lamina is easy to disassemble, so it can be replaced if damaged.

It is a disinfection device in continuous, its use allows to have the cleaning tools free of microorganisms and bacteria all the time. And all that without the disinfecting product being contaminated, since it isn't in contact with the object to disinfect and neither with the microorganisms and bacteria, so the product keeps longer its disinfecting characteristics.

It is ultimately a disinfection device easy, practical, economic and very efficient, that can be used safely in a domestic environment.

## Claims

1. objects disinfection device (3) being such objects sponges, kitchen sponges, cloths, dishcloths, scourer sponges and other cleaning tools, and combs, brushes, toothbrushes, and other personal hygiene tools, **characterized in that** comprises
- a first body (1) formed by a lower Surface (1.1), a lateral surface (1.2) and an upper open surface (1.3), in a manner that define a first chamber (4) suitable to contain a disinfecting product;
- a second body (2) that presents a lower Surface (2.1) comprising a plurality of holes (5), a lateral Surface (2.2) and a upper open area (2.3)
- at least one upper watertight lid (7) arranged on the upper area (2.3) of the second body (2), so between such second body (2) and the at least one upper lid (7), it is defined a second chamber (8) with at least one compartment suitable to contain objects, and;
- one impermeable and breathable lamina (10) of separation between the first chamber (4) and the lower surface (2.1) of the second body (2), fixed to the same with holding means.
- where the second body (2) is placed on the first body (1) and comprises watertight coupling means to the profile of the upper surface (1.3) of the same, being the dimensions of the lower surface (2.1) of the second body (2), smaller or equal than the ones of the upper surface (1.3) of the first body (1).

2. Objects disinfection device (3) according to claim 1, **characterized in that** the coupling means of the second body (2) on the first body (1) are formed by clip elements (12).

3. Objects disinfection device (3), according to claim 2, **characterized in that** comprises furthermore opening and closing means of the second body (2), formed by a first hinged element (6).

4. Objects disinfection device (3) according to any of the previous claims, **characterized in that** when the lower surface (2.1) of the second body (2) has the same dimensions of the upper Surface (1.3) of the first body (1), the fixing means of the lamina (10) are made by a watertight seal arranged in the profile of the lower surface (2.1) of the second body (2).

5. Objects disinfection device (3) according to any of the claims from 1 to 3, **characterized in that** when the lower Surface (2.1) of the second body (2) has smaller dimensions than the ones of the upper Surface (1.3) of the first body (1), such second body (2) comprises a inner lateral surface (11) of connection of its lower surface (2.1) with the coupling means and, the lamina holding means (10) are formed by a watertight seal arranged in the inner lateral surface (11), of the second body (2).

6. Objects disinfection device (3) according to any of the previous claims, **characterized in that** the second chamber (8) comprises at least two separated compartments between them.

7. Objects disinfection device (3) according to claim 6, **characterized in that** each compartment comprises an upper closure lid of the same independent from each other.

8. Objects disinfection device (3) according to any of the previous claims, **characterized in that** at least one upper lid (7) is fixed to the second body (2) with a second hinged element (9).

9. Objects disinfection device (3) according to any of the previous claims, **characterized in that** it comprises a heating device of the disinfecting product made by a resistance and a metallic plate on the same, arranged in a watertight chamber placed on the lower side of the first body, and one connection means of the resistance to a power supply in the lateral of such chamber.

10. Objects disinfection device (3) according to any of the previous claims, **characterized in that** it comprises reload means of disinfecting product formed by an inside pipe that communicates the first chamber (4) of the first body (1) with the second chamber (8) of the second body (2) and a closure cap of the upper ending of such pipe.
